# EUROPÄISCHE PATENTANMELDUNG

(11) **EP 0 554 558 A1**
(43) Veröffentlichungstag der Anmeldung: **11.08.1993**
(21) Anmeldenummer: 92121776.6
(22) Anmeldetag: 22.12.1992
(51) Int. Cl.: A61B 17/32

(54) **Hochdruck-Flüssigkeitsdispensor zur Abgabe von steriler Flüssigkeit**

(30) Priorität: 25.01.1992 DE 4201992
(71) Anmelder: HP MEDICA GESELLSCHAFT mbH FÜR MEDIZINTECHNISCHE SYSTEME, D-86150 Augsburg (DE)
(72) Erfinder: Plechinger, Hans, W-8902 Neusäss (DE); Köhler, Josef, Dr. Ing., W-5100 Aachen (DE)
(74) Vertreter: Vetter, Ewald Otto, Dipl.-Ing.

(57) **Zusammenfassung**

Hochdruck-Flüssigkeitsdispensor zur Abgabe von steriler Flüssigkeit (4) mit so hohem Druck an einen Katheter (6), der in ein Organ (8) eines Menschen oder Tieres eingesetzt ist, daß die Flüssigkeit (4) in Form eines scharfen Flüssigkeitsstrahles (10) aus einer Düse (12) des Katheters (6) austritt, im Organ (8) Organteile oder Fremdkörper abtrennt und/oder zertrümmert und dabei kleine Teilchen bildet, die der Flüssigkeitsstrahl (10) durch den Katheter (6) aus dem Organ (8) heraus abführt. Zur Erzeugung des starken gebündelten Flüssigkeitsstrahles (10) dient ein Vorratsbehälter (34), in welchem die Flüssigkeit (4) für den Flüssigkeitsstrahl (10) gespeichert ist. Der Vorratsbehälter ist in einen Druckbehälter (48) derart eingefügt, daß der Druckbehälter (48) den Druck der Flüssigkeit (4) des Vorratsbehälters (34) aufnimmt, so daß der Druck der Flüssigkeit (4) im Vorratsbehälter (34) wesentlich größer sein kann als ohne Druckbehälter (48), ohne daß der Vorratsbehälter (34) undicht wird oder platzt. Ein Fluid-Stellantrieb (72) bildet zusammen mit einem Pumpenkolben (62) einen Druckverstärker zur Erzeugung eines hohen Druckes der Flüssigkeit (4) im Vorratsbehälter (34) und zum Fördern dieser Flüssigkeit (4) in den Katheter (6) mit dem für den Flüssigkeitsstrahl (10) erforderlichen Druck.

## Beschreibung

Die Erfindung betrifft einen Hochdruck-Flüssigkeitsdispensor zur Abgabe von steriler Flüssigkeit gemäß dem Oberbegriff von Anspruch 1.

In Organen von Menschen und Tieren, beispielsweise Adern, Venen, Leber, Niere, können Gefäßverengungen (z.B. Geschwulste, Kalkablagerungen, Embolien usw.) oder Fremdkörper (Gallensteine, Nierensteine, Geschülste) entstehen. Die Erfinder haben einen Katheter entwickelt, welcher in das zu behandelnde Organ eingesetzt werden kann und mit welchem in dem betreffenden Organ die Gefäßverengung oder der Fremdkörper durch einen scharfen Flüssigkeitsstrahl abgetrennt oder zertrümmert und dabei jeweils in kleine Teilchen zerlegt wird, die von dem Flüssigkeitsstrahl durch den Katheter hindurch aus dem betreffenden Organ abtransportiert werden. Damit der Flüssigkeitsstrahl in dieser Weise wirksam werden kann, muß er einen Druck von mindestens 2 bar haben. Die Druckhöhe ist nach oben nur durch die Druckfestigkeit des Katheters und der Flüssigkeitsleitungen begrenzt und kann beispielsweise bis zu 130 bar betragen. Leitungen, in welchen so hohe Drücke herrschen, sollten sehr kurz sein, wegen der Unfallgefahr bei solchen Drücken. Flüssigkeit mit so hohen Drücken steht in Krankenhäusern nicht zur Verfügung. Zur Erzeugung eines hohen Flüssigkeitsdruckes können Verdichter oder Pumpen verwendet werden. Für den vorgesehenen medizinischen Zweck sind jedoch die bekannten Verdichter oder Pumpen ungeeignet, da die Flüssigkeit vollständig frei von Verunreinigungen, Bakterien und Lufteinschlüssen sein muß, d.h. die Flüssigkeit muß vollständig steril sein. Diese Sterilität ist nicht gegeben, wenn die bekannten Verdichter oder Pumpen verwendet werden, um die Flüssigkeit aus einem Vorratsbehälter zu dem Katheter zu pumpen.

Durch die Erfindung soll die Aufgabe gelöst werden, eine Vorrichtung zu schaffen, mit welcher dem Katheter sterile Flüssigkeit mit einem Druck im Bereich zwischen 2 bar und 130 bar mit der erforderlichen Menge steril und ohne Luftblasen zugeführt werden kann, damit vom Katheter ein scharfer Flüssigkeitsstrahl erzeugt wird, der Flüssigkeitsstrahl in einem menschlichen oder tierischen Organ ein Organteil oder einen Fremdkörper abtrennt und/oder zu kleinen Teilchen zertrümmert und anschließend dieser gleiche Flüssigkeitsstrahl die abgetrennten und zertrümmerten Teilchen durch den Katheter hindurch aus dem Organ herausspült. Die Vorrichtung soll als Massenartikel für eine Vielzahl von Anwendungsmöglichkeiten preiswert hergestellt werden können. Ferner soll die Vorrichtung auf einfache Weise benutzt werden können, ohne daß besonderes Fachpersonal erforderlich ist.

Diese Aufgabe wird gemäß der Erfindung durch die in Anspruch 1 angegebene Kombination von Merkmalen gelöst.

Weitere Merkmale der Erfindung sind in den Unteransprüchen enthalten.

Besondere Merkmale der Erfindung sind: die Verwendung eines vor Benutzung vollständig geschlossenen, die Flüssigkeit steril enthaltenden Vorratsbehälters; Pressung der Flüssigkeit aus dem Vorratsbehälter anstatt Absaugen durch eine Pumpe; Verwendung des in Krankenhäusern üblicherweise zur Verfügung stehenden Druckluftnetzes als Antriebsenergie zum Auspressen der Flüssigkeit aus dem Vorratsbehälter; Verwendung eines Druckwandlers, welcher aus dem niedrigen Druck zwischen 2 und 10 bar der Druckluft einen zweifach bis 30-fach höheren Druck in der sterilen Flüssigkeit des Vorratsbehälters erzeugt; Abstützung des Vorratsbehälters durch einen Druckbehälter entgegen des von der Flüssigkeit auf den Vorratsbehälter wirkenden Druckes derart, daß ein handelsüblicher Vorratsbehälter, beispielsweise in Form einer üblichen Spritze aus Kunststoff, verwendet werden kann, in welchem die Flüssigkeit vor der Benutzung drucklos gespeichert ist, jedoch bei Benutzung der hohe Druck verwendet wird und dieser hohe Druck die elastischen Wände des Vorratsbehälters an die Wände des Druckbehälters preßt und dadurch der hohe Druck der Flüssigkeit im wesentlichen nicht vom Vorratsbehälter, welcher nur als Druckübertragungselement dient, sondern vom Druckbehälter aufgenommen wird, so daß der Vorratsbehälter nicht besonders druckfest zu sein braucht; Regelung des Druckes der Flüssigkeit durch Regelung des Druckes der Druckluft über elektrisch betätigte Druckluftventile durch einen Mikrocomputer; so daß die Flüssigkeit über dem Druckluftteil des Druckwandlers vom elektrischen Teil der Ventile getrennt ist und der Flüssigkeitsdruck in Abhängigkeit von einem vorgegebenen Druck-Sollwert oder einer Druck-Sollwertkurve und einer bestimmten Zeitdauer, die im Mikrocomputer gespeichert sind, oder in Abhängigkeit vom Blutdruck oder Flüssigkeitsdruck in dem behandelten Organ an der Behandlungsstelle von einer den Mikrocomputer enthaltenden Steuereinrichtung geregelt werden kann; Verwendung dieser Vorrichtung zusammen mit einem Katheter, welcher eine Flüssigkeitsstrahl-Düse, eine den Flüssigkeitsstrahl der Düse auffangende Auffan g öffnung, stromab der Auffangöffnung einen kurzen zylindrischen Strömungsberuhigungskanalabschnitt und anschließend einen sich in Strömungsrichtung erweiternden Diffusorabschnitt aufweist, an welchen eine Abführleitung zur Abfuhr des Stromes, welcher aus Flüssigkeit und den von ihm abgetrennten oder zertrümmerten Teilchen aus dem menschlichen oder tierischen Organ, anschließbar ist.

Die Erfindung wird im folgenden mit Bezug auf die Zeichnungen anhand von mehreren bevorzugten Ausführungsformen als Beispiele beschrieben. In den Zeichnungen zeigen
- Fig. 1: eine unmaßstäbliche Darstellung, meistens im Längsschnitt, durch einen Hochdruck-Flüssigkeitsdispensor nach der Erfindung in Anwendung auf einen besonderen Katheter,
- Fig. 2: eine andere Schaltstellung eines in Fig. 1 dargestellten elektrisch betätigbaren Ventils zur Druckluftsteuerung,
- Fig. 3: eine Querschnittsansicht längs der Ebene III-III von Fig. 1, und
- Fig. 4: eine weitere Ausführungsform, teilweise im Längsschnitt, eines Hochdruck-Flüssigkeitsdispensors nach der Erfindung.

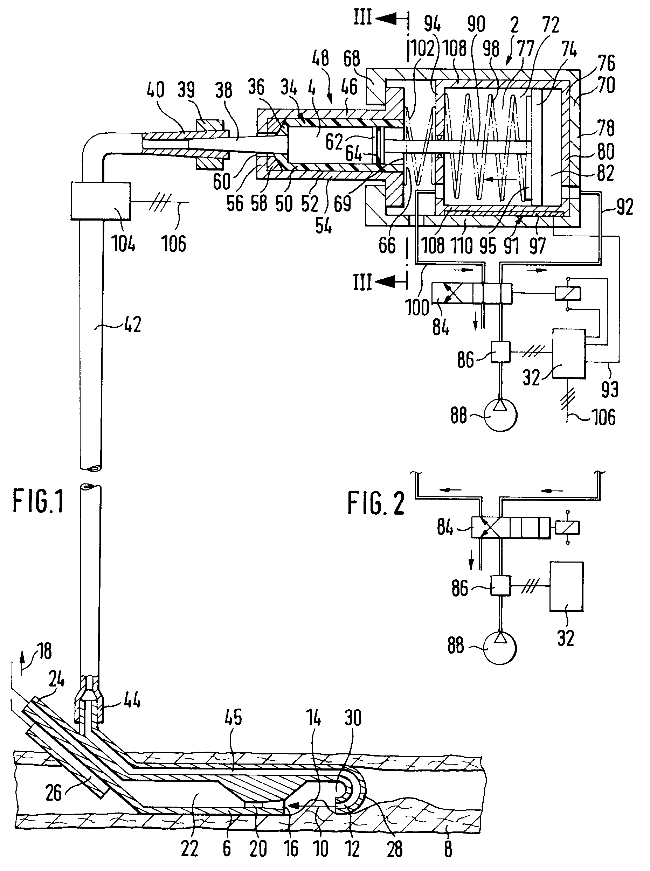

In den Fig. 1, 2 und 3 sind die einzelnen Teile unmaßstäblich dargestellt, damit wesentliche Details deutlicher erkennbar werden. Insbesondere ist in Fig. 1 eine Blutbahn 8, z.B. Ader oder Vene, und ein darin eingesetzter Katheter 6 stark vergrößert dargestellt, während gleichzeitig ein Vorratsbehälter 34 mit steriler Flüssigkeit 4 im Verhältnis zu der Blutbahn in einem stark verkleinerten Maßstab dargestellt ist. Die Größen von Blutbahnen wie Adern und Venen betragen im Durchmesser bekanntlich nur wenige Millimeter. Die Größe des Vorratsbehälters 34 muß in Wirklichkeit so sein, daß er mindestens 1/4 Liter, vorzugsweise aber 1/2 Liter, 1 Liter, 2 Liter, 5 Liter, 10 Liter oder mehr sterile Flüssigkeit enthalten kann. Wenn der Vorratsbehälter 34 kleiner ist, muß er für eine bestimmte Operation zu häufig gegen einen neuen Vorratsbehälter mit steriler Flüssigkeit ausgetauscht werden.

Der in den Fig. 1, 2 und 3 dargestellte Hochdruck-Flüssigkeitsdispensor 2 dient zur Abgabe von steriler Flüssigkeit 4 mit so hohem Druck an einen Katheter 6, der in ein Organ 8 eines Menschen oder Tieres eingesetzt ist, daß die Flüssigkeit 4 in Form eines scharfen Flüssigkeitsstrahles 10 als einer Düse 12 des Katheters 6 austritt. Das Organ 8 kann beispielsweise eine Blutbahn, Herz, Muskel, Leber, Niere, Blase usw. eines Menschen oder Tieres sein. Der scharfe (hoher Druck; kleiner Strahlquerschnitt ohne Streuung) Flüssigkeitsstrahl 10 trennt im Organ 8 Organteile 14 ab und zertrümmert sie (oder bei Fremdkörpern zertrümmert er im Organ 8 die Fremdkörper, z.B. Nierensteine, Gallensteine). Der Flüssigkeitsstrahl 10 reißt die abgetrennten und/oder zertrümmerten Teilchen mit sich in eine Auffangöffnung 16 und führt die Teilchen durch den Katheter 6 hindurch aus dem Organ 8 in Richtung eines Pfeiles 18 heraus. Im Katheter 6 ist stromabwärts der Auffangöffnung 16 nacheinander ein kurzer zylindrischer Strömungsberuhigungskanalabschnitt 20 und anschließend ein in Strömungsrichtung expandierender Diffusorabschnitt 22 und eine sich daran anschließende Abfuhrleitung 24 gebildet. Der Diffusorabschnitt 22 erzeugt an der Auffangöffnung 16 eine verstärkte Saugwirkung, durch welche die vom Flüssigkeitsstrahl 10 abgetrennten oder zertrümmerten Teilchen mit einem starken Sog in den Flüssigkeitsstrahl und mit ihm in die Auffangöffnung 16 gesaugt werden. Ein Drucksensor 26, der vorzugsweise ein Bestandteil des Katheters 6 ist, kann den Druck im behandelten Organ 8 an einer gewünschten Stelle messen, beispielsweise entsprechend Fig. 1 am hinteren Ende des Katheters 6, oder vor dem bogenförmigen, die Düse 12 aufweisenden vorderen Ende 28, oder in einem Zwischenraum 30 des Katheters 6 zwischen dem bogenförmigen vorderen Endteil 28, der Auffangöffnung 16 und dem Flüssigkeitsstrahl 10. Der Sensor 26 gibt ein dem gemessenen Druck entsprechendes elektrisches oder pneumatisches Signal an eine elektronische Steuereinrichtung 32, die vorzugsweise einen Mikroprozessor beinhaltet und den Druck der Flüssigkeit 4 in Abhängigkeit vom gemessenen Druckwert des Sensors 26 regeln kann.

Der Hochdruck-Flüssigkeitsdispensor 2 enthält einen im wesentlichen zylindrischen Vorratsbehälter 34, der die sterile Flüssigkeit 4 enthält und an seiner vorderen Stirnwand 36 mit Leitungsanschlußmitteln 38 versehen ist. Der Vorratsbehälter 34 besteht aus elastischem Material, vorzugsweise aus Kunststoff, und ist vorzugsweise ein handelsüblicher medizinischer Spritzenzylinder, und die Leitungsanschlußmittel 38 bestehen vorzugsweise aus einem bei solchen Spritzenzylindern üblichen, an seiner Oberfläche stromabwärts kegelförmig verjüngten Rohrstück 38 (sogenannter Luer-Kegel, benannt nach dem Erfinder Luer). An die Leitungsanschlußmittel 38 ist das eine Ende 40 einer Fluidleitung 42 angeschlossen, deren anderes Ende 44 an einen zur Düse 12 führenden Zufuhrkanal 45 des Katheters 6 angeschlossen ist. Der Vorratsbehälter 34 ist selbsttragend steif, jedoch leicht flexibel. In der Medizin ist es bekannt, auf das Rohrstück 38 eine Spritzennadel anstelle des Leitungsendes 40 aufzustecken. Vorzugsweise ist eine Haltevorrichtung 39, vorzugsweise ein handelsüblicher Luer-Lock, vorgesehen, welche ein unbeabsichtigtes Trennen der Steckverbindung (Rohrstück 38 mit Leitungsende 40) verhindert. Die Haltevorrichtung 39 kann ein Bajonettverschluß oder eine andere schnell verriegelbare und wieder trennbare Vorrichtung sein. Anstatt eines konischen Rohrstückes 38 kann ein zylindrisches Rohrstück verwendet werden. Wichtig ist, daß es ein in der Medizin handelsübliches Teil ist.

Der Vorratsbehälter 34 ist austauschbar in einen Druckzylinder 46 eines Druckbehälters 48 eingesetzt, welcher den Vorratsbehälter 34 entgegen dem Druck der Flüssigkeit 4 derart abstützt, daß ein wesentlicher Teil des Druckes der Flüssigkeit 4 durch die Wände des Vorratsbehälters 34 hindurch auf die Wände des Druckzylinders 46 übertragen werden. Dadurch kann der Druckwert der Flüssigkeit 4 im Vorratsbehälter 34 um ein Vielfaches höher sein, ohne daß der Vorratsbehälter 34 platzt oder undicht wird, als der Druckwert der Flüssigkeit 4, bei welchem der Vorratsbehälter 34 ohne den Druckzylinder 46 platzen oder undicht werden würde. Zu diesem Zwecke müssen die Stirnwand 36 und die Zylinderwand 50 des Vorratsbehälters 34 flexibel sein und entweder direkt oder über Zwischenelemente an den Wänden des Zylinderraumes 52 des Druckzylinders 46 anliegen. Bei der dargestellten Ausführungsform liegt die Zylinderwand 50 des Vorratsbehälters 34 unmittelbar an der Zylinderwand 54 des Druckzylinders 46 an. Die Stirnwand 36 des Vorratsbehälters 34 ist außen kegelförmig, und die ihr gegenüberliegende Stirnwand 56 des Druckzylinders 46 ist flach. Zur Formanpassung ist zwischen diese beide Stirnwände 36 und 56 ein Druckring 58 eingesetzt, der auf seinen beiden Stirnseiten formmäßig diesen Stirnwänden 36 und 56 angepaßt ist. Dadurch überträgt der Druckring 58 über seine gesamte Querschnittsgröße den Druck der Flüssigkeit 4 vom Vorratsbehälter 34 auf die Stirnwand 56 des Druckzylinders 46. Die Zylinderwand 50 des Vorratsbehälters 34 liegt im wesentlichen über seine gesamte Länge an der Zylinderwand 54 des Zylinderraumes 52 des Druckzylinders 46 an, so daß hier der Druck der Flüssigkeit 4 direkt durch die Zylinderwand 50 des Vorratsbehälters 34 hindurch auf die Zylinderwand 54 des Druckzylinders 46 übertragen wird. Die Spritzenadel 38 erstreckt sich durch zentrale Bohrungen 60 der Druckbehälter-Stirnwand 56 und des Druckringes 58.

Der Vorratsbehälter 34 kann aus einem brüchigen Material wie beispielsweise Glas bestehen. In diesem Falle müssen alle druckübertragenden Flächen zwischen dem Vorratsbehälter 34 und dem Druckzylinder 46 fest aneinander anliegen, damit der Vorratsbehälter 34 bei hohen Drücken der in ihm befindlichen Flüssigkeit 4 nicht springt oder platzt. Dies erschwert aber die erforderliche Austauschbarkeit des Vorratsbehälters 34, wenn er leer ist gegen einen gefüllten neuen Vorratsbehälter 34. Oder es muß, wenn der Vorratsbehälter 34 leer ist, der Vorratsbehälter 34 zusammen mit dem Druckzylinder 46, weil sie unlösbar fest ineinandergefügt sind, ausgetauscht werden. Das ist jedoch teuer. Gemäß der bevorzugten Ausführungsform der Erfindung besteht deshalb der Vorratsbehälter 34 aus Kunststoff. Kunststoff ist, im Verhältnis zu Glas, elastischer. Ein aus Kunststoff bestehender Vorratsbehälter 34 läßt sich auch dann noch leichter axial in den Zylinderraum 52 des Druckzylinders 46 einsetzen, wenn beide den gleichen Durchmesser haben. Ferner kann, um dieses Einsetzen und Auswechseln des Vorratsbehälters zu erleichtern, dieser Vorratsbehälter 34 einen um wenige Hundertstel oder Zehntel mm kleineren Durchmesser haben als der ihn aufnehmende Zylinderraum 52. Dieser geringe Durchmesserunterschied führt bei Kunststoff nicht zu einem Platzen oder Undichtwerden des Vorratsbehälters 34, weil er sich unter dem Druck der Flüssigkeit 4 elastisch ein ganz klein wenig, aber ausreichend für einen festen Druckkontakt mit der Zylinderwand 54 des Druckzylinders 46 und dessen Stirnwand 56 ausdehnen kann. Bei einer solchen Ausdehnung darf der Vorratsbehälter 34 zwischen seiner Zylinderwand 50 und einem in ihm axial geführten Pumpenkolben 62 bezüglich der Flüssigkeit 4 nicht undicht werden. Bei dem Pumpenkolben 62 kann es sich um einen für Spritzen üblichen Kolben handeln, der vorzugsweise mit einer elastischen Ringdichtung 64 versehen ist. Der Pumpenkolben 62 besteht vorzugsweise aus Kunststoff. Der Druckzylinder 46 ist an dem von seiner Stirnwand 56 abgewandten Ende 66 offen, so daß der Vorratsbehälter 34 und der Pumpenkolben 62 durch dieses offene Ende 66 hindurch in den Druckzylinder 46 eingesetzt und, wenn der Vorratsbehälter 34 leer ist, gegen einen mit Flüssigkeit 4 gefüllten neuen Vorratsbehälter 34 und gegebenenfalls auch durch einen anderen Pumpenkolben 62, ausgetauscht werden können.

Am offenen Ende 66 des Druckzylinders 46 ist über einen Schnellverschluß, beispielsweise einen sogenannten Bajonettverschluß 68, ein Druckzylinderkopf 70 des Druckbehälters 48 lösbar befestigt, welcher eine koaxiale Verlängerung des Druckzylinders 46 bildet und einen größeren Durchmesser als dieser hat. Im Druckzylinderkopf 70 ist ein Fluid-Stellantrieb 72 untergebracht, mit welchem der Pumpenkolben 62 in Richtung vom hinteren Ende 69 des Vorratsbehälters 34 bis zu dessen vorderen Stirnwand 36 gedrückt werden kann. Solange der Vorratsbehälter 34 auf Vorrat gehalten wird, liegt der Pumpenkolben 62 an der Flüssigkeit 4 an, ohne einen wesentlichen Druck auf diese Flüssigkeit auszuüben. Wenn bei der Benutzung die Flüssigkeit 4 an den Katheter 6 abgegeben werden soll, drückt der Fluid-Stellantrieb 72 den Pumpenkolben 62 so stark gegen die Flüssigkeit 4 im Vorratsbehälter 34, daß darin ein Druck entsteht, der je nach Anwendungszweck zwischen 2 bar und 130 bar liegen kann. Bei einem niedrigeren Druck dürfte der Flüssigkeitsstrahl 10 im Katheter 6 nicht mehr die gewünschte Wirkung haben. Es können auch Drücke über 130 bar erzeugt werden, jedoch erscheinen höhere Drücke für die genannte Wirkung des Flüssigkeitsstrahles 10 nicht erforderlich zu sein.

Der Fluid-Stellantrieb 72 enthält eine Kolben-Zylindereinheit mit einem Antriebskolben 74 und einem Antriebszylinder 76, in welchem dieser Antriebskolben 74 axial geführt ist. Der Antriebszylinder 76 stützt sich an der vom Schnellverschluß 68 abgewandten hinteren Stirnwand 78 des Druckzylinderkopfes 70 ab und bildet zwischen dem Antriebskolben 74 und der hinteren Stirnwand 80 des Antriebszylinders 76 einen expandierbaren Arbeits-Zylinderraum 82. Der Zylinderraum 82 kann über ein Ventil 84 und einen Druckregler 86 an eine Druckgasquelle 88 angeschlossen werden. Das Druckgas, vorzugsweise Druckluft, der Druckgasquelle 88 drückt den Antriebskolben 74 und den mit ihm über eine Kolbenstange 90 starr verbundenen Pumpenkolben 62 des Vorratsbehälters 34 in Richtung zur vorderen Stirnwand 36 des Vorratsbehälters 34. Erst durch den Druck des Antriebskolbens 74 auf den Pumpenkolben 62 wird im Vorratsbehälter 34 der genannte hohe Druck von 2 bar bis 130 bar der Flüssigkeit 4 erzeugt. Der Antriebskolben 74 und der Antriebszylinder 76 haben einen wesentlich größeren im Druckraum 82 mit Druckluft beaufschlagten wirksamen Querschnitt als der Pumpenkolben 62 und der Vorratsbehälter 34. Dadurch bilden der Antriebskolben 74 und der Antriebszylinder 76 einerseits und der Pumpenkolben 62 und der Vorratsbehälter 34 andererseits zusammen einen Druckwandler, welcher aus einem niedrigen pneumatischen Druck im Druckraum 82 einen zwischen zweimal und dreißigmal höheren Druck in der Flüssigkeit 4 im Vorratsbehälter 34 erzeugt. Dadurch genügt es, wenn der Gasdruck der Druckgasquelle 88 und in deren Leitung 92 zum Druckraum 82 wesentlich niedriger ist und beispielsweise nur 2 bar bis 5 bar beträgt. Damit besteht für den pneumatischen Teil keine oder nur eine geringe Unfallgefahr und es werden keine besonders druckfesten Elemente benötigt.

Wenn der Vorratsbehälter 34 vollständig entleert ist, befindet sich der Pumpenkolben 62 an der vorderen Stirnwand 36 des Vorratsbehälters 34, und der Antriebskolben 74 befindet sich nahe bei einer vorderen Stirnwand 94 des Antriebszylinders 76. Beide Kolben 62 und 74 können durch eine im Antriebszylinder 76 untergebrachte Druckfeder 98 in ihre in Fig. 1 dargestellte Stellungen zurückbewegt werden. Der Zylinderraum 77 zwischen der vorderen Stirnwand 94 des Antriebszylinders 76 und dem Antriebskolben 74 kann in die freie Atmosphäre entlüftet sein, oder durch eine Fluid-Leitung 100 über das Ventil 84 wechselweise entlüftet oder an die Druckgasquelle 88 angeschlossen werden. Fig. 1 zeigt die Position des Ventils 84, bei welcher der Arbeits-Zylinderraum 82 mit Druckluft beaufschlagt und der entgegengesetzte Zylinderraum 77 entlüftet werden. Fig. 2 zeigt das Ventil 84 in einer Position, bei welcher die Druckgasquelle 88 an den Zylinderraum 77 angeschlossen ist und der Arbeits-Druckraum 82 entlüftet wird, so daß der Gasdruck der Druckgasquelle 88 die beiden Kolben 62 und 74 anstelle der Druckfeder 98 in die in Fig. 1 dargestellten Positionen zurückbewegen kann.

Zur Positionierung des Antriebszylinders 76 an der hinteren Stirnwand 78 des Druckzylinderkopfes 70 ist zwischen die vordere Stirnwand 94 des Antriebs-Zylinders 76 und das hintere Ende 66 des Druckzylinders 46 eine Druckfeder 102 eingespannt.

In der elektronischen Steuereinrichtung 32 können bestimmte Druck-Sollwerte der Flüssigkeit 4 gespeichert sein, so daß die elektronische Steuereinrichtung 32 den Druck der Flüssigkeit 4 über den Druckregler 86 in Abhängigkeit von einem Druck-Istwert regeln kann, der im Vorratsbehälter 34 oder der Leitung 42 oder im Katheter 6 gemessen und automatisch der elektronischen Steuereinrichtung 32 mitgeteilt wird. Anstelle eines festen Druck-Sollwertes können in der elektronischen Steuereinrichtung 32 Druck-Sollwertkurven gespeichert sein, in Abhängigkeit von welchen der Druck der Flüssigkeit 4 über bestimmte Zeitperioden verändert und geregelt wird. Unabhängig von diesen beiden Regelungsarten oder zusätzlich zu ihnen kann der Druck der Flüssigkeit 4 oder/und die Fördergeschwindigkeit der Flüssigkeit 4 in Abhängigkeit von dem vom Sensor 26 im Organ 8 gemessenen Organdruck eingestellt oder geregelt werden.

Wenn der Druckzylinderkopf 70 und der Antriebszylinder 76 mindestens teilweise durchsichtig sind, dann ist die jeweilige Position des Antriebskolbens 74 von außen sichtbar. Vorzugsweise ist eine Positionsmeßeinrichtung 91 vorgesehen, welche über elektrische Leitungen 93 durch elektrische Signale die jeweilige axiale Position des Antriebskolbens 74 mit Bezug auf den Antriebszylinder 76 anzeigt. Diese elektrischen Signale können eine optische Anzeige der jeweiligen axialen Position des Antriebskolbens 74 (gleich Position des Pumpenkolbens 62) erzeugen und/oder von der elektronischen Steuereinrichtung 32 zur zeitabhängigen und/oder druckabhängigen (Druck der Behandlungs-Flüssigkeit 4 und/oder Druck im Patienten oder im behandelten Organ 8) Einstellung oder Regelung der Vorschubbewegung des Antriebskolbens 74 und des Pumpenkolbens 62 benutzt werden. Hierfür können in der Steuereinrichtung 32 Sollwert oder Sollwertkurven gespeichert sein. Die Positionsmeßeinrichtung 91 kann aus einem am Antriebskolben 74 befestigten Signalgeber 95, z.B. einem Permanentmagnet-Ring, und einem dessen axiale Position ermittelnden Sensor 97, z.B. einem elektrischen Magnetfeldfühler, bestehen, an welchen die Leitung 93 angschlossen ist; eine sogenannte induktive Wegmeßvorrichtung.

Für bestimmte Anwendungsfälle ist es vorteilhaft, wenn der Flüssigkeitsstrahl 10 kein kontinuierlicher Strahl ist, sondern ein mit hoher Druckfrequenz pulsierender Flüssigkeitsstrahl. Zu diesem Zwecke kann das Druckgas der Druckgasquelle 88 dem Druckraum 82 pulsierend zugeführt werden. Eine bevorzugte Ausführungsform besteht jedoch darin, in die Leitung 42 zwischen dem Vorratsbehälter 34 und dem Katheter 6 einen Pulsator 104 einzusetzen, welcher von der elektronischen Steuereinrichtung 32 über elektrische Leitungen 106 gesteuert wird und dem Druck der Flüssigkeit 4 in der Leitung 42 eine Druckpulsation überlagert. Diese "Überlagerung" kann dadurch erfolgen, daß der Pulsator 104 den Strömungsquerschnitt der Leitung 42 an einer bestimmten Stelle mit der gewünschten Pulsationsfrequenz verengt oder verschließt und wieder öffnet.

Das Ventil 84 für den Fluid-Stellantrieb 72 wird von der elektronischen Steuereinrichtung 32 vorzugsweise elektrisch betätigt.

Der Druckbehälter 48, der Fluid-Stellantrieb 72 und der Vorratsbehälter 34 bilden zusammen eine handliche Baueinheit. Zum Austauschen des Vorratsbehälters 34 gegen einen gefüllten anderen brauchen lediglich der Druckzylinder 46 und der Zylinderkopf 70 des Druckbehälters 48 am Schnellverschluß 68 voneinander getrennt zu werden. Sie bestehen vorzugsweise aus Metall. Im getrennten Zustand kann der Vorratsbehälter 34 aus dem Druckzylinder 46 durch sein offenes hinteres Ende 66 herausgezogen werden. Falls erforderlich, kann auf diese Weise auch der Druckring 58 ausgetauscht werden. Der Antriebszylinder 76 kann aus Metall oder Kunststoff bestehen. Der Antriebszylinder 76 kann ähnlich wie der Vorratsbehälter 34 aus einem Material oder aus so dünnen Wänden bestehen, daß er den im Druckraum 82 erzeugten Pneumatikdruck nur aushält, ohne undicht zu werden oder zu platzen, wenn er entsprechend Fig. 1 mit seiner hinteren Stirnwand 80 und mit seiner Zylinderwand 108 an der hinteren Stirnwand 78 und der Zylinderwand 110 des Druckzylinderkopfes 70 anliegt, so daß letzterer den Druck aus dem Druckraum 82 aufnimmt und die betreffenden Wände des Antriebszylinders 76 nur druckübertragende Zwischenelemente sind.

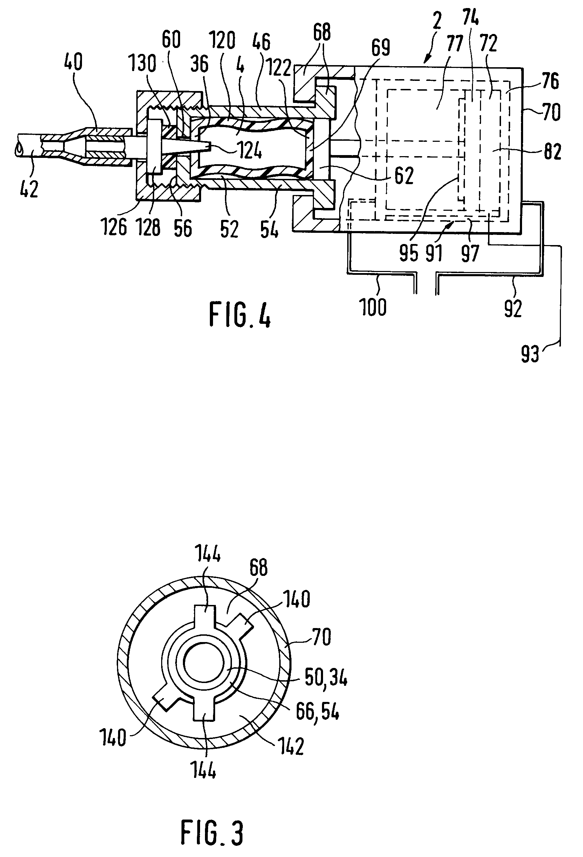

Fig. 3 zeigt eine mögliche Ausführungsform für den Schnellverschluß 68. Gemäß dieser Ausführungsform kann der Schnellverschluß durch radiale Ausnehmungen 140 in der vorderen Stirnwand 142 des Druckzylinderkopfes 70 einerseits, und durch radiale Vorsprünge 144 am hinteren Ende 66 des Druckzylinders 46 gebildet sein (oder umgekehrt). Die radialen Vorsprünge 144 können durch die radialen Öffnungen 140 axial hindurchgeführt und dann so weit relativ zu diesen Öffnungen verdreht werden, daß sie an den danebenliegenden Bereichen der Stirnwand 142 des Druckzylinderkopfes 70 axial anliegen.

Alle Teile des Vorratsbehälters 34 und des Druckzylinders 46 haben vorzugsweise eine kreisrunde Querschnittsform.

Bei der Ausführungsform nach Fig. 1 dient der Vorratsbehälter 34 als Pumpenzylinder, in welchem der Pumpenkolben 62 flüssigkeitsdicht axial geführt ist. Im unbenutzten Zustand ist die Flüssigkeit 4 im Vorratsbehälter 34 steril gefüllt, jedoch im wesentlichen drucklos, und der Pumpenkolben 62 dient als Verschlußelement des Vorratsbehälters 34.

Bei der in Fig. 4 dargestellten weiteren Ausführungsform eines Hochdruck-Flüssigkeitsdispensors sind alle Teile gleich wie in Fig. 1, mit folgender Ausnahme: Der Vorratsbehälter 120 für die Flüssigkeit 4 ist kein selbsttragend starrer Körper, sondern ein durch den auf seine hintere Stirnwand 122 drückenden Pumpenkolben 62 zusammenfaltbarer Behälter. Die vordere Stirnwand 36 dieses faltbaren Behälters, der vorzugsweise aus Kunststoff besteht, kann mit einer Spritzennadel 38 entsprechend Fig. 1 versehen sein oder als Leitungs-Anschlußmittel so ausgebildet sein, daß er von einer Hohlnadel 124 durchstochen werden kann, die mit dem stromaufwärtigen Ende 40 der Leitung 42 verbunden ist. Zu diesem Zwecke kann eine Überwurfmutter 126 auf die Zylinderwand 54 aufgeschraubt sein, welche die Hohlnadel 124 mittels eines Ringbunds 128 durch die vordere Stirnwand 36 hindurchdrückt. Zwischen dem Ringbund 128 und der vorderen Stirnwand 56 des Druckzylinders 46 befindet sich vorzugsweise eine die Bohrung 60 abdichtende Dichtung 130. Der Pumpenkolben 62 ist im Druckzylinder 46 axial geführt und liegt an dessen Zylinderwand 54 verschiebbar, jedoch fluiddicht an.

Bei der Ausführungsform nach Fig. 4 dient der Druckzylinder 46 als Pumpenzylinder, in welchem der Pumpenkolben 62 flüssigkeitsdicht axial geführt ist.

In abgewandelter Ausführungsform könnte anstelle der Druckgas liefernden Druckgasquelle 88 eine Flüssigkeitsdruckquelle 88 vorgesehen sein, welche eine Flüssigkeit mit einem Druck zwischen 2 bar und 10 bar in die Arbeits-Druckkammer 82 des Fluid-Stellantriebes 72 liefert, in welcher die Druckflüssigkeit auf den Antriebskolben 74 wirkt und über den Pumpenkolben 62 die Flüssigkeit 4 im Vorratsbehälter 34 auf einen Druck zwischen etwa 6 bar und 130 bar verdichtet.

Gemäß einer bevorzugten Ausführungsform nach der Erfindung wird die Flüssigkeit 4 mit einem Druck im Bereich von 48 bar (ungefähr 600 p.s.i.) und 60 bar (ungefähr 900 p.s.i.) von dem Pumpenkolben 62 aus dem Vorratsbehälter 34 in den Katheter 6 getrieben.

Die Verwendung eines Druckbehälters 48 hat den Vorteil, daß der Vorratsbehälter 34 nicht besonders druckfest zu sein braucht und deshalb billig hergestellt werden kann, vorzugsweise aus Kunststoff; daß er als Massenartikel in großer Stückzahl sterilisiert mit der Flüssigkeit gefüllt und mit dieser Flüssigkeit transportiert und gelagert werden kann, wobei die Flüssigkeit im Vorratsbehälter drucklos sein kann oder einen Unterdruck oder einen nur geringen Überdruck haben kann; daß der Vorratsbehälter 34 eine handelsübliche medizinische Spritzen-Kartusche sein kann; daß der Druckbehälter 48 nacheinander für eine Vielzahl von Vorratsbehältern 34 benutzt werden kann.

Wenn eine Druck-Fluid-Qelle 28 zur Verfügung steht, welche Druck-Fluid mit einem ausreichend hohen Druck liefert, brauchen der Antriebskolben 74 und der Pumpenkolben 62 zusammen keinen Druckverstärker zu bilden. In diesem Falle kann die Querschnittsfläche des Antriebskolbens 74 gleich groß oder kleiner sein als die Querschnittsfläche des Pumpenkolbens 62. Der Nachteil hierbei ist, daß die Druck-Fluid-Leitung 92 und ihre Leitungsanschlüsse für den hohen Druck druckfest ausgebildet sein müssen. In allen Fällen kann das Druck-Fluid Druck-Flüssigkeit sein, ist aber vorzugsweise Druckluft. Eine hohe Betriebssicherheit ohne Gefahr, daß das Druck-Fluid in die sterile Flüssigkeit 4 im Vorratsbehälter 34 gelangt, ist dadurch gegeben, daß das Druck-Fluid der Druck-Fluid-Quelle 88 nicht nur durch den Pumpenkolben 62, sondern auch durch den Antriebskolben 74 von der sterilen Flüssigkeit 4 getrennt ist.

## Patentansprüche

1. Hochdruck-Flüssigkeitsdispensor zur Abgabe von steriler Flüssigkeit (4) mit so hohem Druck an einen Katheter (6), der in ein Organ (8) eines Menschen oder Tieres eingesetzt ist, daß die Flüssigkeit (4) in Form eines scharfen Flüssigkeitsstrahles (10) aus einer Düse (12) des Katheters (6) austritt, im Organ (8) Organteile, Ablagerungen oder Fremdkörper abtrennt und/oder zertrümmert und dabei kleine Teilchen bildet, und dann unter Mitnahme dieser Teilchen über eine Auffangöffnung (16) des Katheters (6) in den Katheter zurückströmt und durch den Katheter hindurch aus dem Organ herausströmt,
dadurch **gekennzeichnet**, daß
die Flüssigkeit (4) in einem Vorratsbehälter (34) steril gespeichert ist, welcher in einen Druckbehälter (48) austauschbar eingesetzt ist;
daß der Druckbehälter (48) einen durch Druck-Fluid betätigbaren Pumpenkolben (62) zum Ausdrücken der sterilen Flüssigkeit (4) mit dem hohen Druck aus dem Vorratsbehälter (34) in den Katheter (6) enthält;
daß der Vorratsbehälter (34) auf allen Seiten, mit Ausnahme im Bereich des Pumpenkolbens (62), vom Druckbehälter (48) derart abgestützt wird, daß der Druckbehälter (48) den hohen Druck vom Vorratsbehälter (34) aufnimmt, welchen der Pumpenkolben (62) in der sterilen Flüssigkeit (4) im Vorratsbehälter (34) erzeugt;
daß die Innendruck-Festigkeit des Vorratsbehälter (34) wesentlich kleiner ist als die Innendruck-Festigkeit des Druckbehälters (48);
daß die sterile Flüssigkeit (4) im Vorratsbehälter (34) drucklos oder mit einem bestimmten Unterdruck oder einem betimmten niedrigen Überdruck gespeichert ist, welcher wesentlich niedriger als der vom Pumpenkolben (62) in ihr erzeugte Druck ist.

2. Hochdruck-Flüssigkeitsdispensor nach Anspruch 1,
**gekennzeichnet durch**
folgende Merkmale:
2.1. einen im wesentlichen zylindrischen Vorratsbehälter (34), der die sterile Flüssigkeit (4) im unbenutzten Zustand im wesentlichen drucklos speichert und an seinem vorderen Ende Leitungsanschlußmittel (38) zur Abgabe der Flüssigkeit mit einem hohen Flüssigkeitsdruck an den Katheter (6) aufweist;
2.2. einen Druckbehälter (48), in welchen der Vorratsbehälter (34) austauschbar eingesetzt ist und welcher den Vorratsbehälter (34) entgegen dem Druck der Flüssigkeit derart abstützt, daß der Vorratsbehälter (34) nur als Druckübertragungselement wirksam ist, auf welches von innen der hohe Druck der Flüssigkeit (4) und von außen im wesentlichen der gleiche Gegendruck durch den Druckbehälter (46) wirkt und dadurch der Druckwert der Flüssigkeit (4) im Vorratsbehälter (34) ein Vielfaches höher sein kann, ohne daß der Vorratsbehälter (34) platzt oder undicht wird, als der Druckwert, bei welchem der Vorratsbehälter (34) ohne den Druckbehälter (46) platzen oder undicht werden würde;
2.3. einen Pumpenkolben (62), der in Richtung vom hinteren Ende (69) zum vorderen Ende (36) des Vorratsbehälters (34) axial bewegbar ist und mit einer bestimmten Kolbenquerschnittsgröße die Flüssigkeit (4) gegen das vordere Ende (36) drängt;
2.4. einen Fluid-Stellantrieb (72), der eine Kolben-Zylindereinheit mit einem Antriebszylinder (76) und einem darin axial verschiebbaren Antriebskolben (74) aufweist, von welchem der bewegliche Teil (Antriebskolben oder Antriebszylinder) auf den Pumpenkolben (62) in der genannten Richtung wirkt und von welchen der ortsfeste Teil (Antriebszylinder oder Antriebskolben) entgegen der genannten Richtung auf den Druckbehälter (46) wirkt;
2.5. der Antriebskolben (74) und der Pumpenkolben (62) bilden einen Druckverstärker, bei welchem die von einem Druckfluid beaufschlagte Kolbenquerschnittsgröße des Antriebskolbens (74) wesentlich größer ist als die auf die sterile Flüssigkeit (4) wirkende Kolbenquerschnittsgröße des Pumpenkolbens (62), so daß aus einem bestimmten niedrigen Fluiddruckwert von beispielsweise 2 bar bis 10 bar im Fluid-Stellantrieb (72) ein zweifach bis 30-fach höherer Flüssigkeitsdruck in der sterilen Flüssigkeit (4) im Vorratsbehälter (34) erzeugt wird.

3. Hochdruck-Flüssigkeitsdispensor nach Anspruch 1 oder 2,
**dadurch gekennzeichnet,**
daß das Fluid zur Betätigung des Fluid-Stellantriebes (72) Druckluft ist.

4. Hochdruck-Flüssigkeitsdispensor nach einem der Ansprüche 1 bis 3,
**dadurch gekennzeichnet,**
daß die Fluidzufuhr zum Stellantrieb (72) durch mindestens ein elektrisch betätigtes Ventil (84) und eine elektronische Steuereinrichtung (32) mit einem Mikrocomputer geregelt wird.

5. Hochdruck-Flüssigkeitsdispensor nach einem der Ansprüche 1 bis 4,
**dadurch gekennzeichnet,**
daß der Fluid-Stellantrieb (72) im Druckbehälter (48) untergebracht ist und zusammen mit diesem eine Baueinheit bildet.

6. Hochdruck-Flüssigkeitsdispensor nach einem der Ansprüche 1 bis 5,
**dadurch gekennzeichnet,**
daß ein Schnellverschluß (68) zum Öffnen des Druckbehälters (48) für den Austausch des Vorratsbehälters (34) vorgesehen ist.

7. Hochdruck-Flüssigkeitsdispensor nach einem der Ansprüche 1 bis 6,
**dadurch gekennzeichnet,**
daß zwischen eine vordere Stirnwand (36) des Vorratsbehälters (34) und eine ihr gegenüberliegende vordere Stirnwand (56) des Druckbehälters (48) eine Druckplatte (58) eingesetzt ist, deren Oberflächenformen an die Formen der beiden Stirnwände angepaßt sind, so daß die Stirnflächen dieser Wände und der Druckplatte jeweils über ihre gesamte Ausdehnung aneinander anliegen.

8. Hochdruck-Flüssigkeitsdispensor nach einem der Ansprüche 1 bis 7,
**dadurch gekennzeichnet,**
daß der Druckbehälter (48) eine um ein Vielfaches höhere Druckfestigkeit hat als der Vorratsbehälter (34).

9. Hochdruck-Flüssigkeitsdispensor nach einem der Ansprüche 1 bis 8,
**dadurch gekennzeichnet,**
daß der Vorratsbehälter (34) aus Kunststoff besteht.

10. Hochdruck-Flüssigkeitsdispensor nach einem der Ansprüche 1 bis 9,
**dadurch gekennzeichnet,**
daß der Vorratsbehälter (34) durch den Spritzenzylinder einer handelsüblichen medizinischen Spritze gebildet ist.

11. Hochdruck-Flüssigkeitsdispensor nach einem der Ansprüche 1 bis 10,
**dadurch gekennzeichnet,**
daß der Pumpenkolben (62) ein handelsüblicher medizinischer Spritzenkolben ist, der im Vorratsbehälter (34) druckdicht an einer Zylinderwand anliegt und darin axial verschiebbar ist.

12. Hochdruck-Flüssigkeitsdispensor nach einem der Ansprüche 1 bis 11,
**dadurch gekennzeichnet,**
daß die Leitungs-Anschlußmittel (38) ein handelsübliches hohles, außen konisches Rohrstück (38), dessen Kanal durch die vordere Stirnwand (36) hindurch mit dem Innenraum des Spritzenzylinders (34) in Strömungsverbindung steht, und eine Verriegelungsvorrichtung (39) zur Haltesicherung eines auf das Rohrstück (38) aufgesteckten Leitungsendes (40) aufweisen.

13. Hochdruck-Flüssigkeitsdispensor nach einem der Ansprüche 1 bis 12,
**dadurch gekennzeichnet,**
daß der Druckbehälter (46) aus Metall besteht.

14. Hochdruck-Flüssigkeitsdispensor nach einem der Ansprüche 1 bis 13,
**dadurch gekennzeichnet,**
daß Mittel (104) zur Druckpulsation der vom Vorratsbehälter (34) abgegebenen sterilen Flüssigkeit (4) vorgesehen sind.

15. Hochdruck-Flüssigkeitsdispensor nach einem der Ansprüche 1 bis 14,
**dadurch gekennzeichnet,**
daß der Vorratsbehälter (120) ein axial zusammenfaltbarer Beutel aus Kunststoff ist, der mit der sterilen Flüssigkeit (4) gefüllt ist und an Innenflächen eines Zylinderraumes (52) des Druckbehälters (48) druckübertragend anliegt, und daß der Pumpenkolben (62) im Zylinderraum (52) des Druckbehälters (48) untergebracht ist und von dessen Zylinderinnenflächen axial geführt wird und axial gegen das hintere Ende (122) des flexiblen Flüssigkeitsbeutels (120) drückt.
